# EUROPEAN PATENT SPECIFICATION

(11) **EP 2 221 024 B1**
(45) Date of publication and mention of the grant of the patent: **05.10.2011**
(21) Application number: 10150798.6
(22) Date of filing: 14.01.2010
(51) Int. Cl.: A61F 2/16

(54) **Intraocular lens configured to offset optical effects caused by optic deformation**
Intraokularlinse, die zum Kompensieren von optischen Effekten, die von optischer Verformung verursacht werden, eingerichtet ist
Lentille intra-oculaire prévue pour compenser les effets optiques provoqués par déformation optique

(30) Priority: 19.02.2009 US 153869 P; 23.12.2009 US 646328
(43) Date of publication of application: 25.08.2010
(73) Proprietor: ALCON RESEARCH, LTD., 76134-2099 TX, Texas Fort Worth (US)
(72) Inventor: Stanley, Daniel Walter, Midlothian, TX 76065 (US); Wensrich, Douglas Brent, Bedford, TX 76021 (US); Angelopoulos, Robert Dimitri, Fort Worth, TX 76109 (US)
(74) Representative: Hanna, Peter William Derek

(56) References cited:
- EP-A1- 1 932 492
- WO-A1-92/03989
- WO-A1-2006/025726
- WO-A1-2007/040964
- WO-A1-2008/091152
- WO-A2-2008/077795
- US-A1- 2007 100 444

## Description

### TECHNICAL FIELD OF THE INVENTION

The present invention relates to intraocular lenses. More particularly, the present invention relates to intraocular lenses with haptics and methods for offsetting the optical effects caused by optic deformation.

### BACKGROUND OF THE INVENTION

The human eye is a generally spherical body defined by an outer wall called the sclera, having a transparent bulbous front portion called the cornea. The lens of the human eye is located within the generally spherical body, behind the cornea. The iris is located between the lens and the cornea, dividing the eye into an anterior chamber in front of the iris and a posterior chamber in back of the iris. A central opening in the iris, called the pupil, controls the amount of light that reaches the lens. Light is refracted by the cornea and by the lens onto the retina at the rear of the eye. The lens is a bi-convex, highly transparent structure surrounded by a thin lens capsule. The lens capsule is supported at its periphery by suspensory ligaments called zonules, which are continuous with the ciliary muscle. The focal length of the lens is changed by the ciliary muscle pulling and releasing the zonules. Just in front of the zonules, between the ciliary muscle and iris, is a region referred to as the ciliary sulcus.

A cataract condition results when the material of the lens becomes clouded, thereby obstructing the passage of light. To correct this condition, three alternative forms of surgery are generally used, known as intracapsular extraction, extracapsular extraction, and phacoemulsification. In intracapsular cataract extraction, the zonules around the entire periphery of the lens capsule are severed, and the entire lens structure, including the lens capsule, is then removed. In extracapsular cataract extraction and phacoemulsification, only the clouded material within the lens capsule is removed, while the transparent posterior lens capsule wall with its peripheral portion, as well as the zonules, are left in place in the eye.

Intracapsular extraction, extracapsular extraction, and phacoemulsification eliminate the light blockage due to the cataract condition. The light entering the eye, however, is thereafter defocused due to the lack of a lens. A contact lens can be placed on the exterior surface of the eye, but this approach has the disadvantage that the patient has virtually no useful sight when the contact lens is removed. A preferred alternative is to implant an artificial lens, known as an intraocular lens (IOL), directly within the eye. An intraocular lens generally comprises a disk-shaped, transparent lens optic and two curved attachment arms referred to as haptics. The lens is implanted through an incision made near the periphery of the cornea, which may be the same incision as is used to remove the cataract. An intraocular lens may be implanted in either the anterior chamber of the eye, in front of the iris, or in the posterior chamber, behind the iris.

An anterior chamber lens is supported by contact of the haptics with a corner, or angle, of the anterior chamber which is formed by the union of the iris and the cornea. In the case of a posterior chamber lens, there are two alternative techniques of support. In the first technique, the intraocular lens and its haptics are placed in the sack-like structure formed by the intact posterior and peripheral walls of the lens capsule. The haptics are compressed slightly against the periphery of the lens capsule and thereby hold the intraocular lens in place. In the second technique, the intraocular lens is placed in front of and outside the lens capsule. The haptics are sandwiched between the iris and the zonules, in the region of the ciliary sulcus, to hold the lens in place.

During implantation, an intraocular lens can become compressed by the haptics.

This can lead to the lens shape becoming distorted; thereby impacting the intended optical quality of the lens.

WO 2006/025726 and EP 1 932 492 disclose methods to design lenses to reduce aberrations, but do not account for aberrations produced when the optical surfaces are deformed by compression when implanted.

The document WO 2007/040964 A1 discloses an intraocular lens optic having a rigid and a deformable surface, wherein these surfaces are configured to reduce an optical aberration when the lens is compressed by ocular forces.

### SUMMARY OF THE INVENTION

The present invention provides a lens optic, an introcular lens and methods, in accordance with claims which follows. Traditional IOL designs have not considered the optical effect of the deformation induced by the mechanical elements that fixate the IOL in the eye. The deformation caused by compression may create aberrations (e.g., astigmatism, coma, etc) in the lens optic that can reduce the optical performance of the lens, especially at larger pupil diameters. The effects of optical surface deformation become more important as lens optics become more precise. Embodiments disclosed herein incorporate a lens optic designed with features, such as surface geometry, refractive index or other features for negating aberrations induced when the lens is in the eye. For example, the lens geometry can be selected geometry in an uncompressed state so that the lens has a desired geometry in a compressed state that is that reduces or eliminates the optical effects of compression in typical IOLs. As another example, the outer geometry of the lens can be selected so that the refractive index of the material is less than or greater the refractive index of the rest of the IOL to produce desired results when the IOL is implanted.)

Embodiments of a lens optic may include a first surface having a first surface uncompressed geometry in an uncompressed state and a second surface having a first geometry in an uncompressed state. At least one of the first surface uncompressed geometry and the second surface uncompressed geometry may be formed such that the lens optic is substantially free of optical effects when in the compressed state. In some embodiments, the compressed state is due to compression of the lens optic when positioned in an eye compartment. The second geometry may be due to compressive forces exerted by one or more haptics on the lens optic. The lens optic can further comprise an aberration selected to correct astigmatism. In some embodiments, the aberration is based on an anticipated compression of 0.5 mm to 1.0 mm. In some embodiments, the aberration is selected to correct coma. In some embodiments, the aberration is selected to correct a spherical aberration.

An embodiment of an intraocular lens may include a lens optic and a pair of haptics coupled to the lens optic. The lens optic may have a first surface having a first surface uncompressed geometry in an uncompressed state and a second surface having a second surface uncompressed geometry in an uncompressed state. At least one of the first surface uncompressed geometry and the second surface uncompressed geometry may be formed such that the lens optic is substantially free of optical effects when in the compressed state. In some embodiments, the haptics define a first axis on the lens optic between the haptics and a second axis may be defined on the lens optic at some angle relative to the first axis. The uncompressed geometry of one or more of the first surface and the second surface relative to the first axis may differ from the compressed geometry of the first surface or the second surface about the second axis. The lens optic may have a thinner edge thickness where the edge intersects the second axis than where the edge intersects the first axis. In some embodiments, the uncompressed geometry of one or more of the first surface and the second surface is based on an anticipated compression of the lens optic due to the eye compartment or the haptics so that the lens optic compresses to a desired shape when implanted.

Embodiments disclosed herein may also be directed to a method of offsetting an optical effect due to deformation of a lens optic. The method may include the steps of identifying an aberration in the eye for which correction is desired, determining an expected amount of compression caused by implanting an intraocular lens into a chamber and selecting an intraocular lens for implantation based on the expected compression. The first surface has a first surface uncompressed geometry and the second surface has a second surface uncompressed geometry when in an uncompressed state. At least one of the first surface and the second surface has a compressed geometry when in a compressed state. The intraocular lens may comprise a lens optic with a first surface and a second surface and a pair of haptics. The first geometry of one or more of the first surface and the second surface may be formed such that the lens optic is substantially free of optical effects when in the compressed state. One or more of the first surface and the second surface may have a first geometry when in an uncompressed state and one or more of the first surface and the second surface may have a second geometry when in a compressed state.

Additionally, the method can include creating one or more aberrations on one or more of the first surface and the second surface. The aberrations created in the intraocular lens when the lens is in an uncompressed state may offset the optical effects caused by the compression of the lens optic. In some embodiments, the aberration includes one of astigmatism, coma, or spherical aberration. In some embodiments, the aberration is formed to about 0.17 D at the spectacle place and being up to about 0.25 D at the intraocular lens plane. In some embodiments, the haptics define a first axis on the lens optic between the haptics, a second axis is defined at an angle relative to the first axis. The uncompressed geometry differs from the desired geometry about the second axis.

In some embodiments, determining the amount of compression caused by implanting the intraocular lens into the chamber comprises estimating an amount of compression of the lens optic attributable to compression by the haptics, wherein creating one or more aberrations on one or more of the first surface and the second surface comprises selecting the desired geometry to account for the amount of compression attributable to compression by the haptics. In some embodiments, the method includes forming the lens optic having one or more of the first surface and the second surface with an aspheric curve.

One advantage to creating a lens having a surface geometry based on an anticipated compressed state may be the ability to create thinner lenses. Thus, instead of making the lens optic thicker to reduce the effect of compression, or reduce the stiffness of the haptics, which could affect how well the IOL remains in place once implanted, embodiments disclosed herein can allow thinner lenses to be implanted without sacrificing optical performance.

### BRIEF DESCRIPTION OF THE FIGURES

A more complete understanding of the present invention and the advantages thereof may be acquired by referring to the following description, taken in conjunction with the accompanying drawings in which like reference numbers indicate like features.
FIGURE 1 is a perspective view of one embodiment of an intraocular lens;
FIGURE 2 depicts a schematic diagram of one embodiment of an intraocular lens having a convex surface, illustrating axial displacements for the lens;
FIGURE 3 depicts a schematic diagram of one embodiment of an intraocular lens having a convex surface, illustrating axial displacements for the lens with average rigid-body motion removed;
FIGURE 4 depicts a schematic diagram of one embodiment of an intraocular lens having a concave surface, illustrating axial displacements for the lens;
FIGURE 5 depicts a schematic diagram of one embodiment of an intraocular lens having a concave surface, illustrating axial displacements for the lens with average rigid-body motion removed;
FIGURE 6 depicts a schematic diagram of theoretical spherical performance of a lens in a model eye;
FIGURE 7 depicts a spot diagram corresponding to the model eye depicted in FIGURE 6;
FIGURE 8 depicts a diagram of the Modulation Transform Function (MTF) for the model eye depicted in FIGURE 6;
FIGURE 9 depicts a spot diagram representing the optical performance of an eye showing signs of astigmatism;
FIGURE 10 depicts an MTF diagram showing phakic performance of an exemplary eye showing signs of astigmatism;
FIGURE 11 depicts a spot diagram representing the optical performance of an eye having an intraocular lens positioned therein to correct for astigmatism;
FIGURE 12 depicts an MTF diagram showing phakic performance of an exemplary eye having an intraocular lens positioned therein to correct for astigmatism;
FIGURE 13 depicts a flow chart of one method for improving the optical performance of an intraocular lens;
FIGURES 14A and 14B depict views of one embodiment of a lens optic, illustrating an axis where compression force is applied a force axis;
FIGURE 15A depicts a top view of one embodiment of a lens optic;
FIGURES 15B and 15C side views of one embodiment of a lens optic;
FIGURE 15D depicts a close-up of an aspheric optic side view of the lens optic depicted in FIGURE 15C;
FIGURE 16A depicts a perspective view of one embodiment of a lens having an aberration for offsetting the optical effects due to compression;
FIGURE 16B depicts a cutaway side view along the force axis of the lens depicted in FIGURE 16A;
FIGURE 16C depicts a cutaway side view along the bending axis of the lens depicted in FIGURE 16A;
FIGURE 17A depicts a perspective view of one embodiment of a lens having an aberration for offsetting the optical effects due to compression;
FIGURE 17B depicts a cutaway side view along the force axis of the lens depicted in FIGURE 17A; and
FIGURE 17C depicts a cutaway side view along the bending axis of the lens depicted in FIGURE 17A.

### DETAILED DESCRIPTION

Embodiments of a method and apparatus for offsetting the optical effects caused by compression of the lens and deformation induced by compression of the lens optic or fixation components are disclosed.

Various embodiments of the disclosure are illustrated in the FIGURES, like numerals being generally used to refer to like and corresponding parts of the various drawings.

As used herein, the terms "comprises," "comprising," "includes," "including," "has," "having" or any other variation thereof, are intended to cover a non-exclusive inclusion. For example, a process, article, or apparatus that comprises a list of elements is not necessarily limited to only those elements but may include other elements not expressly listed or inherent to such process, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Additionally, any examples or illustrations given herein are not to be regarded in any way as restrictions on, limits to, or express definitions of, any term or terms with which they are utilized. Instead, these examples or illustrations are to be regarded as being described with respect to one particular embodiment and as illustrative only. Those of ordinary skill in the art will appreciate that any term or terms with which these examples or illustrations are utilized will encompass other embodiments which may or may not be given therewith or elsewhere in the specification and all such embodiments are intended to be included within the scope of that term or terms. Language designating such nonlimiting examples and illustrations includes, but is not limited to: "for example", "for instance", "e.g.", "in one embodiment".

Embodiments of methods and systems disclosed herein may be used to offset one or more optical effects caused by deformation of the lens optic.

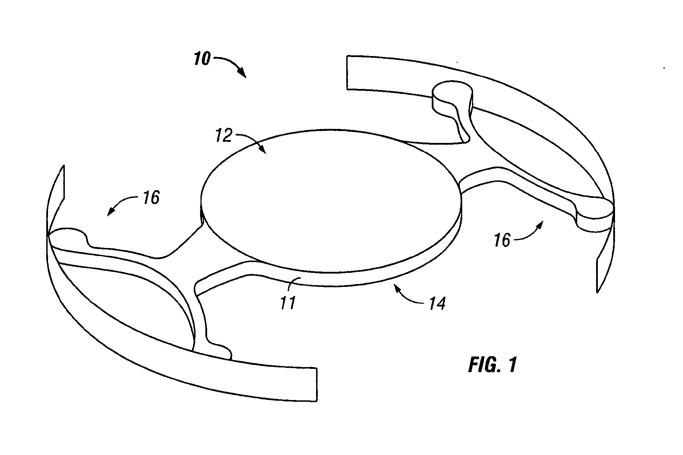

FIGURE 1 depicts a perspective view of one embodiment of intraocular lens 10 comprising optic 11 having surfaces 12 and 14 and haptics 16. In some embodiments, surfaces 12 and 14 may be convex or concave (e.g., lens optic 11 may have a convex surface 12 and a concave surface 14 or some other configuration). In some embodiments, lens 10 may be formed with each haptic 16 formed from one or more elements. For example, FIGURE 1 depicts haptics 16 with a single wide portion near optic 11, a bifurcated section distal the wide portion, and a curved outer portion connected to both ends of the bifurcated section. Those skilled in the art will appreciate that other designs for haptics are possible, with each design introducing a unique compressive force on optic 11.

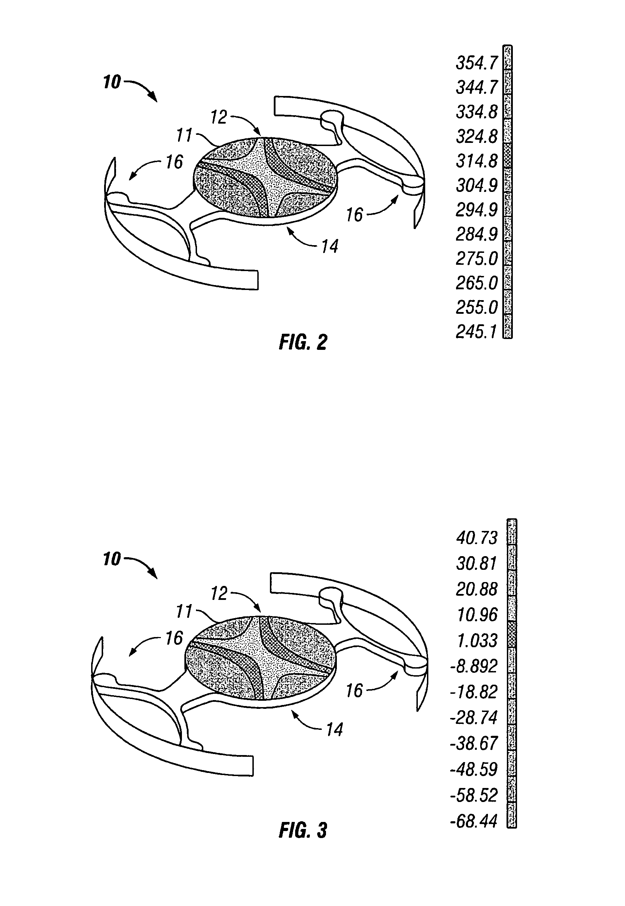

FIGURE 2 depicts a schematic diagram of one embodiment of intraocular lens 10 depicted in FIGURE 1 and optic 11 having convex surface 12. FIGURE 2 depicts one embodiment of lens 10 showing axial displacements of convex surface 12. FIGURE 3 depicts a schematic diagram of the embodiment of intraocular lens 10 depicted in FIGURE 2, with the average rigid-body motion removed. By removing the effects of average rigid-body motion from lens 10, substantially all the axial displacement of optic 11 may be due to elastic deformation of optic 11.

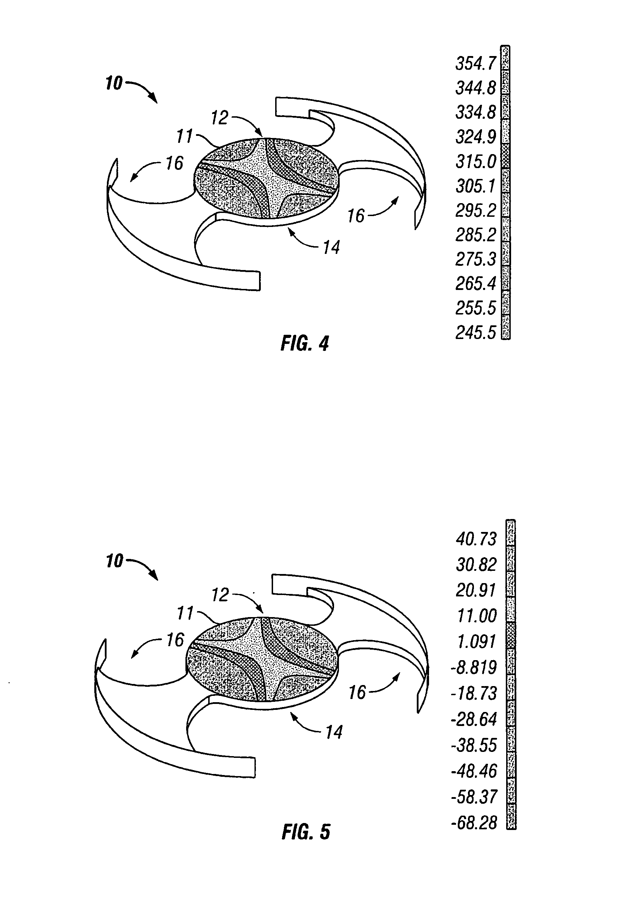

FIGURE 4 depicts a schematic diagram of one embodiment of intraocular lens 10 depicted in FIGURE 1 with optic 11 having concave surface 12. FIGURE 4 depicts one embodiment of lens 10 showing axial displacements of concave surface 12. FIGURE 5 depicts a schematic diagram of the embodiment of intraocular lens 10 depicted in FIGURE 4, with the average rigid-body motion removed. By removing the average rigid-body motion, substantially all the axial displacement of optic 11 associated with concave surface 12 may be due to elastic deformation of optic 11.

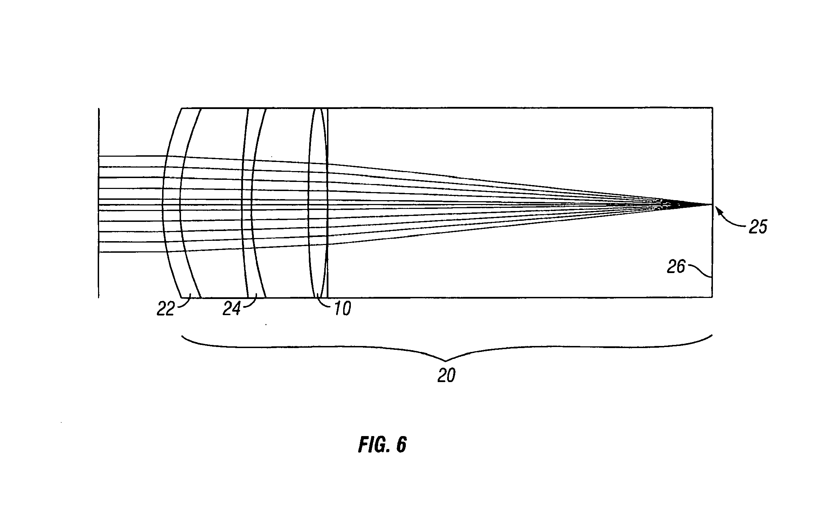

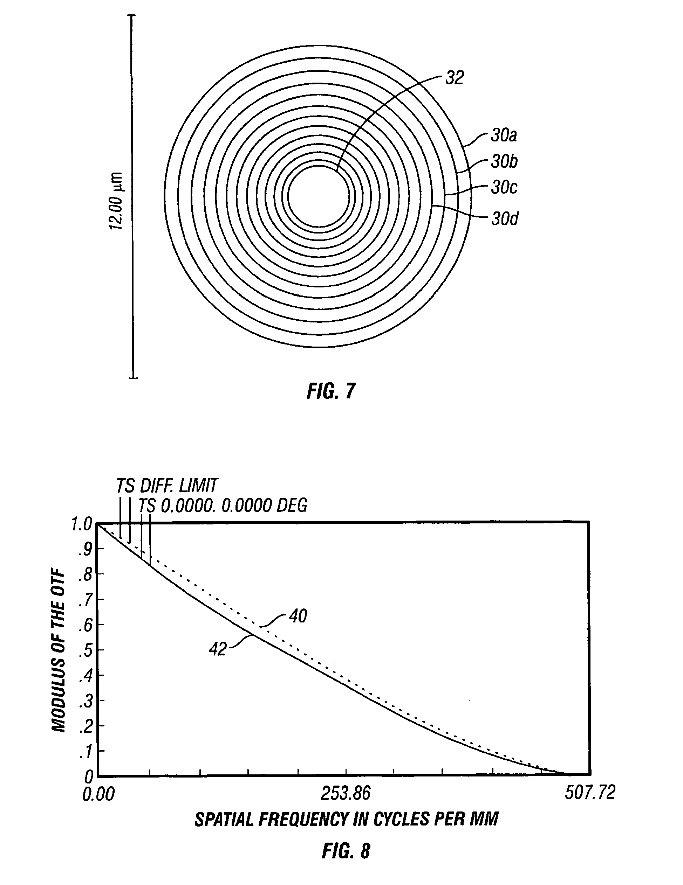

FIGURE 6 depicts a schematic diagram of theoretical spherical performance of lens 10 in a model eye. Light entering model eye 20 (i.e. from the bottom as depicted in FIGURE 6) passes through spherical cornea 22, phakic lens 10 and lens 24 such that the light is ideally focused at a single spot 25 on retinal plate 26 some distance from lens 10. FIGURE 7 depicts a spot diagram corresponding to model eye 20 depicted in FIGURE 6. Light focused by a nominal system would produce a very small spot, as shown in FIGURE 7. The rings in the spot diagram are residual spherical aberration, which will be present in any spherical optical system, the case of a nominal system the spherical aberration would be on the order of 1/25 of a wave. FIGURE 8 depicts a diagram of the Modulation Transfer Function (MTF) for model eye 20, in which the modulus of the optical transfer function (which is normalized to be between 0 and 1) for a range of spatial frequencies (in cycles per mm). Theoretical curve 40 represents the diffraction limit of the modulus of the optical transfer function for a range of spatial frequencies between 0 and about 508 cycles per mm. Actual curve 42 represents the actual modulus of the optical transfer function for the same range of spatial frequencies. Ideally, actual curve 42 for model eye 20 would equal theoretical curve 40 for all points.

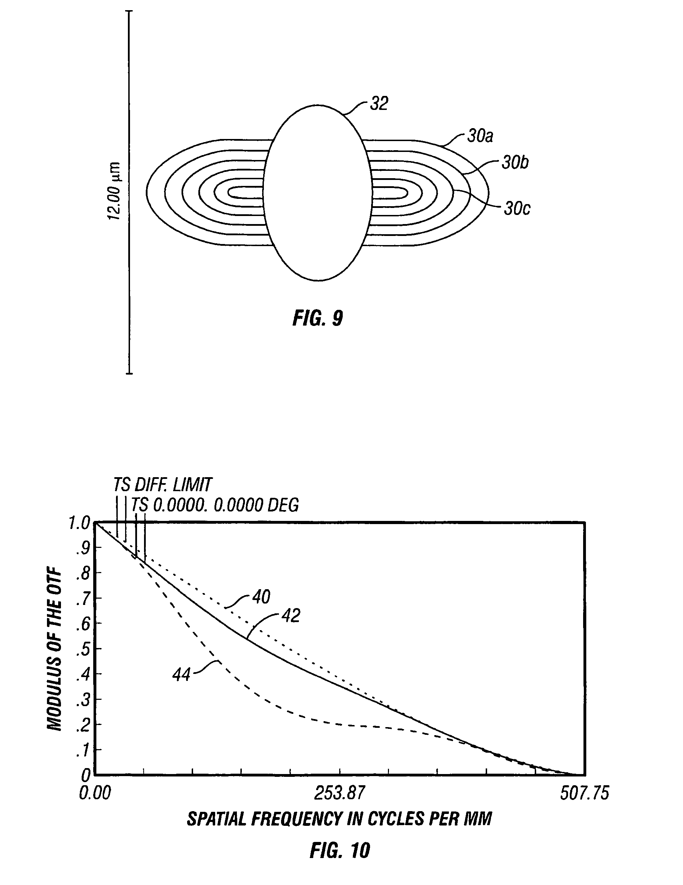

FIGURE 9 depicts a spot diagram representing the optical performance of an eye with a deformed intraocular lens 10. Instead of spot diagram 30 forming concentric circles, rings 30 have an irregular shape similar to an oval or some other non-circular shape depicting the introduction of astigmatism. Astigmatism is only selected as an example for demonstration purposes.
FIGURE 10 depicts a diagram of the performance of an exemplary eye showing signs of astigmatism for a range of spatial frequencies (in cycles per mm) in an eye with a deformed phakic as described in conjunction with FIGURE 9. Curve 40 depicts the normalized theoretical modulus of the optical transfer function for a range of spatial frequencies. Curve 42 represents the normalized expected modulus of the optical transfer function for a range of spatial frequencies. Curve 44 represents the normalized actual modulus of the optical transfer function for a range of spatial frequencies. Thus, the effect due to the lens may contribute heavily to the poor optical performance.

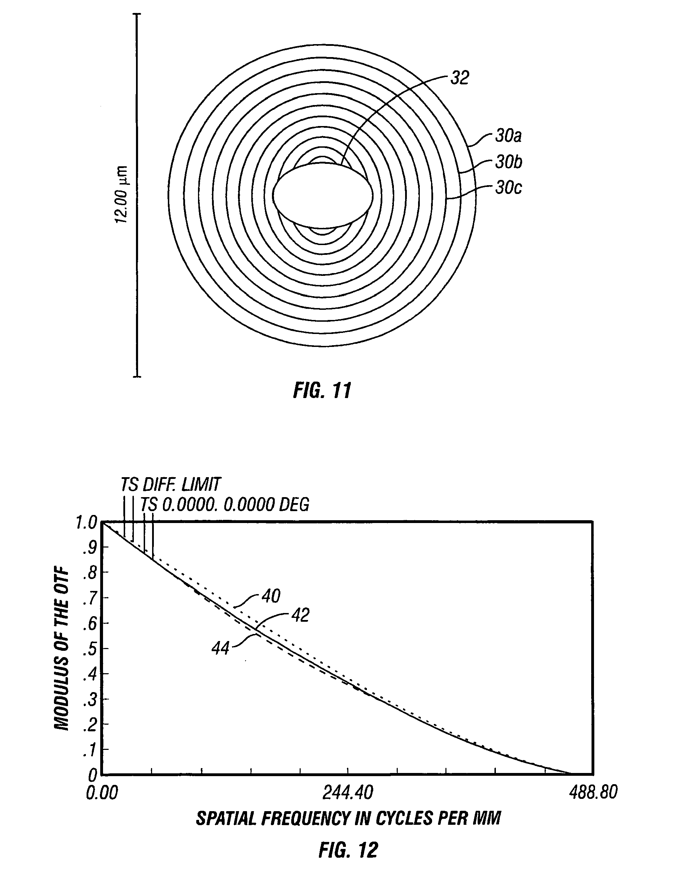

FIGURE 11 depicts a spot diagram representing the optical performance of an eye having a spectacle lens. The spectacle lens may focus to form a spot 32 that is more circular than spot 32 depicted in FIGURE 9. Those skilled in the art will appreciate that the spot diagram depicted in FIGURE 11 represents an improved optical performance of an eye as compared to the spot diagram depicted in FIGURE 9.
FIGURE 12 depicts a diagram of the phakic performance of an exemplary eye having a spectacle lens for a range of spatial frequencies (in cycles per mm). Curve 40 depicts the normalized theoretical modulus of the optical transfer function over the range of spatial frequencies. Curve 42 represents the normalized expected modulus of the optical transfer function over the range of spatial frequencies. Curve 44 represents the normalized actual modulus of the optical transfer function over the range of spatial frequencies. Curve 44 more closely approximated curve 42. Those skilled in the art will appreciate that the MTF diagram depicted in FIGURE 12 represents an improved optical performance of an eye than the MTF diagram depicted in FIGURE 10.

In the above case, the spectacle lens improves performance for the reformed phakic. Another method for improving the optical performance of an intraocular lens involves offsetting the optical effects due to deformation of the lens optic. Deformation of the lens optic may be due to compression of the lens optic, which is typically the result of implanting the IOL in the capsular bag or the anterior or posterior chambers of the ciliary sulcus. Deformation of the lens optic may also be due to compression of the haptics. Deformation may also be caused by a combination of lens optic compression and the effect of haptic compression on the lens optic. Various features of the IOL, such as geometry, material, optical properties or other features of the lens optic or overall IOL, can be selected so that the lens optic is substantially free of optical effects when in its compressed state.

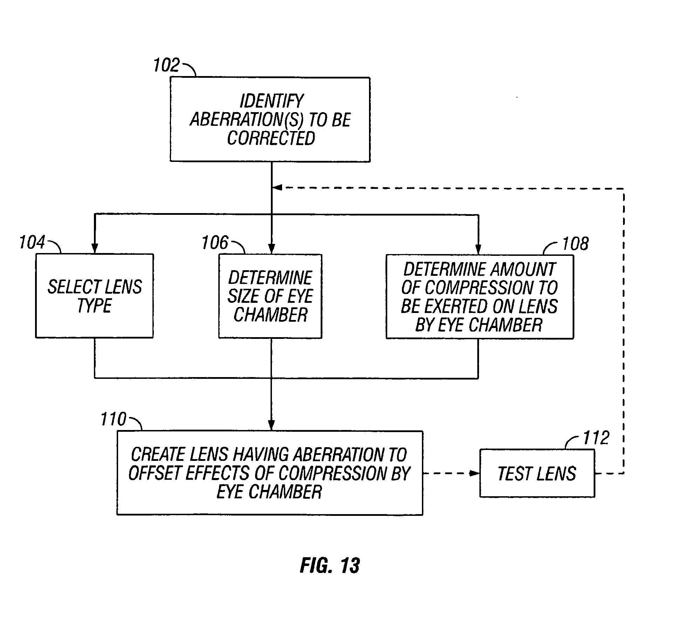

FIGURE 13 depicts flow chart 100 of one method for improving the optical performance of intraocular lens 10. In step 102, one or more aberrations of the eye is identified for correction. Aberrations of the eye include bias, tilt, power (defocus), astigmatism, coma, spherical and trefoil, as well as higher orders of astigmatism, coma and sphericity, and may also include pentafoil, tetrafoil, higher order spherical aberrations and others.

In step 104, lens 10 may be selected for placement in the eye. Lens 10 may be selected based on the material used to manufacture lens 10. Those skilled in the art will appreciate that each lens material may have a unique set of material properties, such as Young's modulus, bulk modulus, shear modulus, and the like. In some embodiments, lens 10 may be manufactured from a soft plastic material. In one embodiment, an AcrySof® lens manufactured by Alcon Labs of Fort Worth, Texas may be selected.

In step 106, the eye chamber into which lens 10 is to be implanted may be measured. In some embodiments, measuring may include measuring a diameter. Measuring the diameter may be necessary because the chamber has an associated amount of variation. For example, it may be necessary to measure the diameter of the anterior chamber of the ciliary sulcus prior to implantation of lens 10 because the anterior chamber has a relatively large variation in diameter. Measuring the eye chamber may also include other measurements.

Those skilled in the art will appreciate that steps 104 and 106 may be performed in either order. That is, the eye chamber into which lens 10 is to be implanted may be measured before lens 10 is selected or lens 10 may be selected before the eye chamber is measured. For example, the pupil diameter, size of the eye chamber, or some other characteristic of the eye or eye chamber may affect the type of IOL 10 to be used in the eye.

In step 108, the amount of expected compression exerted on lens optic 11 by the chamber may be determined. Determining the amount of compression may involve predicting the compression due to the difference between the outer diameter of lens optic 11 and the inner diameter of the eye chamber. In some embodiments, determining the amount of compression may involve determining an expected range of compression. The compression in the anterior chamber may range from about 0.5 mm to about 1.0 mm.

In some embodiments, step 108 of determining the amount of compression on lens optic 11 may involve predicting the compression due to one or more characteristics of haptics 16. In some embodiments, one or more of the thickness, diameter, shape or length of haptics 16 may affect the amount of compression exerted on lens optic 11. In some embodiments, the angle of connection of haptics 16 to lens optic 11, the location of the attachment point, the means by which haptics 16 are connected to lens optic 11, the area formed at the attachment point, or some other characteristic of how haptics 16 are coupled to lens optic 11 may affect the compression of lens optic 11 in the eye chamber.

In some embodiments, one or more numerical programs, finite element analysis (FEA), ray-tracing or other methods may be used to predict the deformation of lens optic 11. In one embodiment, lens optics 11 having different geometries or aberrations may be positioned in various model eyes and spot diagrams or MTF diagrams may be generated to predict the deformation of lens optic 11.

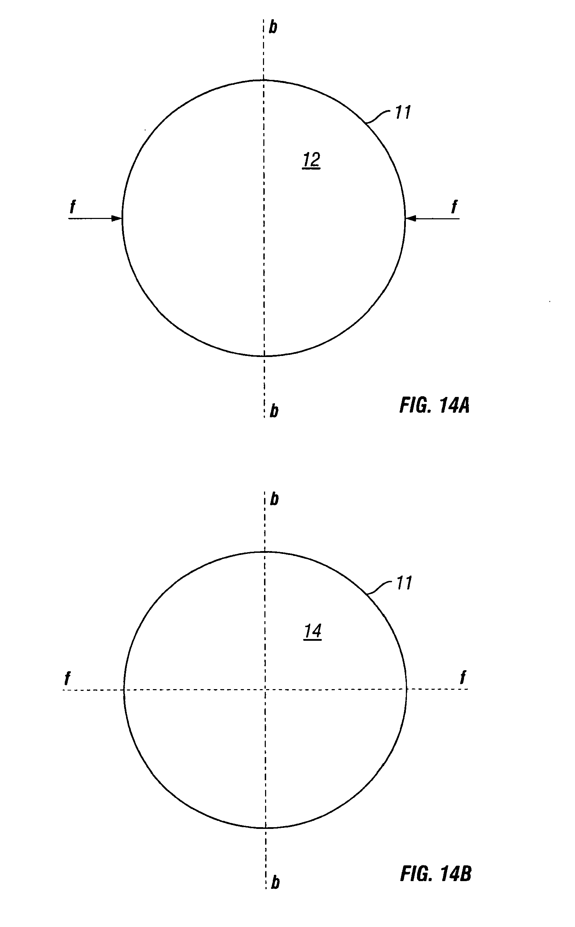

In step 110, intraocular lens 10 may be created such that when lens 10 is implanted in the eye chamber, lens optic 11 will deform into a desired compressed geometry. Intraocular lens 10 may be created to correct for astigmatism, coma, spherical aberration, or some other deformation. Intraocular lens 10 may be created to correct for a higher-order deformation. Creating lens 10 may include determining the amount of deformation that needs to be introduced into lens 10 to offset the optical effects of compression of the lens optic or compression of the haptics or both. In some embodiments, determining the amount of deformation that needs to be introduced to offset an optical effect includes identifying a bending axis. Turning briefly to FIGURE 14A, this figure depicts a perspective view of one embodiment of optic 11 having bending axis b-b. Bending axis b-b may be the result of the construction of optic 11 or the application of forces F on optic 11 or some combination. Bending of optic 11 may impart an aberration onto optic 11 of lens 10. FIGURE 14B depicts a top view of one embodiment of optic 11, showing bending axis b-b and force axis f-f. In some embodiments, force axis f-f is perpendicular to bending axis b-b. The angle and/or value of forces F applied on optic 11 may determine the type and value of an aberration on surface 12 or 14 of optic 11.

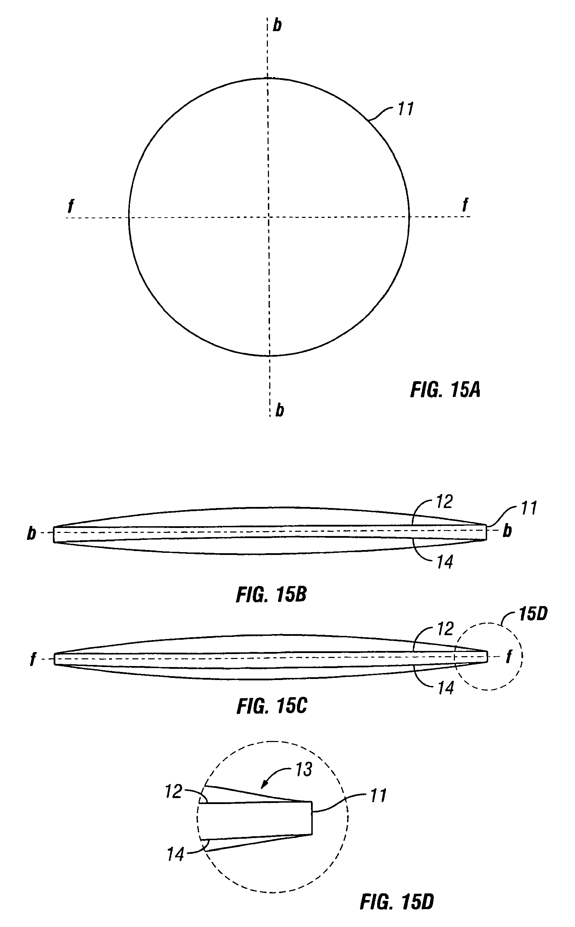

Embodiments of intraocular lens 10 may be created such that any corrective deformation is introduced on posterior surface 12 and/or anterior surface 14 of lens optic 11. Aberrations on surface 12 and/or surface 14 may be symmetric or asymmetric to offset an effect of compression. FIGURE 15A depicts one embodiment of optic 11 with bending axis b-b perpendicular to force axis f-f. FIGURES 15B and 15C depict a side view of optic 11 depicted in FIGURE 15A. FIGURE 15B depicts a cutaway side view along axis f-f and FIGURE 15C depicts a side view along axis b-b. Surface 12 contains an aspheric component in this embodiment. Figure 15B depicts a side view of the lens perpendicular to the force axis and shows how the edge of the optic of a toric lens thins to accommodate the steep axis. Figure 15C depicts a side view of the lens perpendicular to the bending axis. An aspheric curve may be useful for focusing light and may be formed on either surface 12 or 14 or both. FIGURE 15D depicts a close-up of an aspheric optic side view. As shown in FIGURES 15A-15D, embodiments of optic 11 may have surface 12 or 14 with a constant profile, may be symmetric about an axis, may have regions of differing profiles, or some combination thereof.

The force applied on axis f-f may determine the effect on optical performance of optic 11. The curvature, shape, thickness or other characteristic of surface 14 or 12 of optic 11 may be based on a force F applied to optic 11. Thus, for the same correction but anticipating different compression rates, different lenses 10 may be created. In some embodiments, a set of lenses 10 for a patient may be created having different surfaces 12 and 14. For example, a first lens 10 may be created based on an anticipated compression of 0.5 mm and a second lens may be created based on an anticipated compression of 1.0 mm. A set of lenses may be created based on an expected deformation. For example, a first lens 10 may have surface 12 with a selected thickness and a second lens 10 may have surface 12 with a selected thickness. In some embodiments, a set of lenses 10 may be created having aberrations on surface 12, surface 14, or some combination.

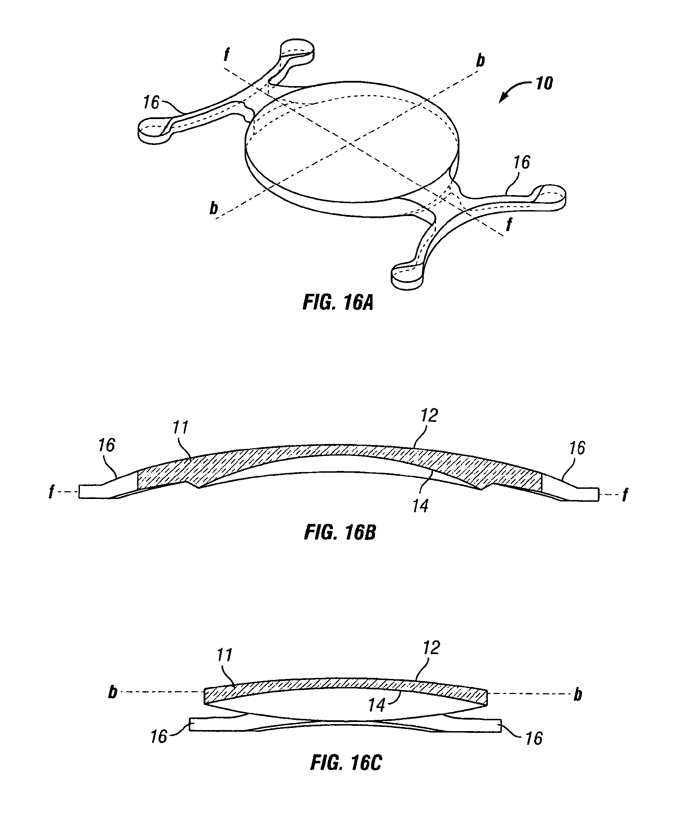

FIGURE 16A depicts a perspective view of one embodiment of lens 10 having optic 11 and haptics 16, showing surface 12. In FIGURE 16A, bending axis b-b is shown perpendicular to force axis f-f defined by haptics 16. However, bending axis b-b and force axis f-f are not so constrained, and each may depend on the construction of optic 11, haptics 16, or lens 10. FIGURE 16A further depicts optic 11 having a thinner cross-section at the edge near bending axis b-b as compared to the area near force axis f-f. FIGURE 16B depicts a cutaway side view along force axis f-f (i.e., perpendicular to bending axis b-b) of lens 10 depicted in FIGURE 16A. FIGURE 16C depicts a cutaway side view along axis b-b (i.e., perpendicular to axis f-f) of lens 10 depicted in FIGURE 16A. The uncompressed geometries of surfaces 12 and 14 of lenses 10 depicted in FIGURES 16A-16C may be compressed during positioning in the eye compartment to offset the deformation such that lens 10 provides a desired optical performance.

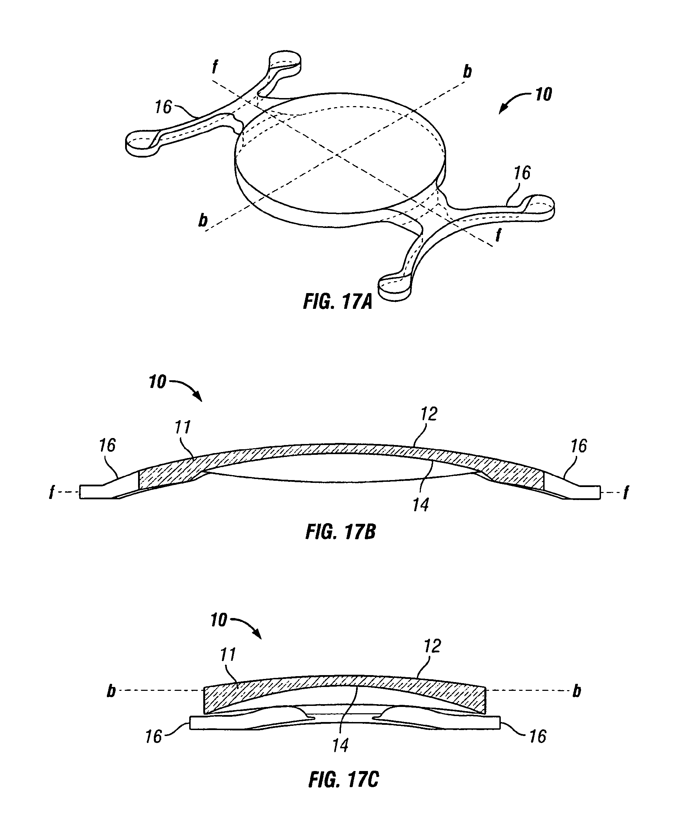

FIGURE 17A depicts a perspective view of one embodiment of lens 10 having optic 11 and haptics 16. In FIGURE 17A, bending axis b-b is shown perpendicular to force axis f-f defined by haptics 16. However, bending axis b-b and force axis f-f are not so constrained, and each may depend on the construction of optic 11, haptics 16, or lens 10. FIGURE 17A further depicts optic 11 having a thicker cross-section near bending axis b-b as compared to the area near force axis f-f. FIGURE 17B depicts a cutaway side view along force axis f-f (i.e., perpendicular to bending axis b-b) of lens 10 depicted in FIGURE 17A. FIGURE 17C depicts a cutaway side view along force axis f-f (i.e., perpendicular to bending axis b-b) of lens 10 depicted in FIGURE 17A. The uncompressed geometries of surfaces 12 and 14 of lenses 10 depicted in FIGURES 17A-17C may be compressed during positioning in the eye compartment to offset the deformation such that lens 10 provides a desired optical performance.

A comparison of optic 11 for FIGURE 16C and 17C shows that although surface 12 for may be substantially the same along either axis, surface 14 may have a steeper curve such that the outer edges of optic 11 are thicker. Thus, optic 11 depicted in FIGURE 16B may be thicker near the edges than optic 11 depicted in FIGURE 17B but optic 11 (as depicted in FIGURE 16C) may be thinner around the edge than optic 11 (as depicted in FIGURE 17C). Those skilled in the art will appreciate that the thickness of optic 11, the position or orientation of bending axis b-b and force axis f-f, and aberrations may be formed on either surface 12 or 14 or both of optic 11, may be symmetric or asymmetric with respect to an axis or surface, and may be used in combination for offsetting the effects of compression on optic 11. The edge thickness of lens optic 11 can follow a pattern such as a sine wave with the thickest portions at the force axis and the thinnest portions at the bending axis. The edge of lens optic 11 can also follow other patterns and need not be symmetric.

Returning briefly to FIGURE 10, in some embodiments, a method for improving the optical performance of an intraocular lens may include step 112 of testing the lens. Testing the lens may involve placing lens 10 in a model eye and testing the optical performance. Testing lens 10 may involve placing lens 10 in the eye and testing the optical performance. Other testing may be possible to ensure deformed lens 10 provides a desired optical performance.

In some embodiments of a method for implanting lens 10 in an eye chamber, a set of lenses may be created to correct an aberration and each lens 10 may be created based on a predicted compression. During surgery, the surgeon may implant a first lens 10 having a first uncompressed geometry and then determine if lens 10 adequately corrects the aberration. If lens 10 does not correct the aberration, the surgeon may remove lens 10 and try a larger or smaller lens until a desired lens 10 is implanted in the eye compartment.

Although embodiments have been described in detail herein, it should be understood that the description is by way of example only and is not to be construed in a limiting sense. It is to be further understood, therefore, that numerous changes in the details of the embodiments and additional embodiments will be apparent to, and may be made by, persons of ordinary skill in the art having reference to this description. It is contemplated that all such changes and additional embodiments are within scope of the claims below and their legal equivalents.

## Claims

1. A lens optic (11) for use in an intraocular lens, comprising:
a first surface (12) having a first surface uncompressed geometry in an uncompressed state; and
a second surface (14) having a second surface uncompressed geometry in an uncompressed state,
wherein at least one of the first surface and the second surface has a compressed geometry when in a compressed state, wherein the first surface uncompressed geometry and the second surface uncompressed geometry are selected to at least partially offset optical effects, **characterized in that** the optical effects are caused by the expected compression of the lens caused by implanting the lens into an eye chamber.

2. The lens optic of claim 1, wherein the compressed geometry is due to compressive forces exerted by one or more haptics on the lens optic.

3. The lens optic of claim 1, wherein the lens optic (11) comprises an aberration.

4. The lens optic of claim 3, wherein the aberration is selected to correct astigmatism.

5. The lens optic of claim 3, wherein the aberration is based on an anticipated compression of 0.5 mm to 1.0 mm.

6. The lens optic of claim 3, wherein the aberration is selected to correct coma.

7. The lens optic of claim 3, wherein the aberration is selected to correct at least one of a group comprising bias, tilt, astigmatism, coma, spherical aberration, trefoil, higher orders of astigmatism, coma and sphericity, pentafoil, tetrafoil, and higher order spherical aberrations.

8. An intraocular lens (10) for offsetting the optical effects due to compressive deformation, comprising the lens optic of any one of claims 1 to 7, and a pair of haptics (16) coupled to the lens optic.

9. The intraocular lens of claim 8, wherein the haptics (16) define a first axis on the lens optic between the haptics, wherein a second axis on the lens optic is at some angle relative to the first axis, wherein the lens optic has a thinner edge thickness where the edge intersects the second axis than where the edge intersects the first axis.

10. The intraocular lens of claim 8, wherein the first axis comprises a force axis (f-f) and the second axis comprises a bending axis (b-b).

11. A method of offsetting an optical effect due to deformation of a lens optic, comprising:
identifying (102) an aberration in the eye for which correction is desired;
determining (108) an expected amount of compression caused by implanting an intraocular lens into an eye chamber; and
configuring the intraocular lens to have a first surface (12) and a second surface (14),
wherein the first surface has a first surface uncompressed geometry and the second surface has a second surface uncompressed geometry when in an uncompressed state, wherein at least one of the first surface and the second surface has a compressed geometry when in a compressed state, wherein the first surface uncompressed geometry and the second surface uncompressed geometry are selected to at least partially offset the optical effects caused by the expected compression of the lens.

12. The method of claim 11, further comprising creating an aberration on at least one of the first surface (12) or the second surface (14) to correct one of astigmatism, coma, or spherical aberration.

13. The method of claim 11, wherein haptics (16) define a first axis on the lens optic between the haptics, the lens optic defining a second axis at an angle relative to the first axis, the uncompressed geometry differing from a desired geometry about the second axis.

14. The method of claim 11, wherein the aberration is formed to about 0.17 D at the spectacle place and being up to about 0.25 D at the intraocular lens plane.

15. The method of claim 11, wherein determining (108) the expected amount of compression caused by implanting the intraocular lens into the chamber comprises estimating an amount of compression of the lens optic attributable to compression by the haptics (16).

16. The method of claim 11, further comprising forming the lens optic having one or more of the first surface (12) and the second surface (14) with an aspheric curve.

## Patentansprüche

1. Linsenoptik (11) zur Verwendung in einer intraokularen Linse, aufweisend:
eine erste Oberfläche (12) mit einer nicht komprimierten Geometrie der ersten Oberfläche in einem nicht komprimierten Zustand; und
eine zweite Oberfläche (14) mit einer nicht komprimierten Geometrie der zweiten Oberfläche in einem nicht komprimierten Zustand;
wobei wenigstens eine von der ersten Oberfläche und der zweiten Oberfläche eine komprimierte Geometrie hat, wenn sie sich in einem komprimierten Zustand befindet, wobei die nicht komprimierte Geometrie der ersten Oberfläche und die nicht komprimierte Geometrie der zweiten Oberfläche so gewählt sind, dass sie wenigstens teilweise optische Effekte kompensieren, **dadurch gekennzeichnet, dass** die optischen Effekte durch die erwartete Kompression der Linse verursacht werden, die durch die Implantation der Linse in eine Augenkammer verursacht wird.

2. Linsenoptik nach Anspruch 1, wobei die komprimierte Geometrie auf Druckkräften beruht, die von einem oder mehreren Haltebügeln auf die Linsenoptik ausgeübt werden.

3. Linsenoptik nach Anspruch 1, wobei die Linsenoptik (11) eine Aberration aufweist.

4. Linsenoptik nach Anspruch 3, wobei die Aberration zur Korrektur von Astigmatismus ausgewählt ist.

5. Linsenoptik nach Anspruch 3, wobei die Aberration auf einer angenommenen Kompression von 0,5 mm bis 1,0 mm basiert.

6. Linsenoptik nach Anspruch 3, wobei die Aberration zur Korrektur von Coma ausgewählt ist.

7. Linsenoptik nach Anspruch 3, wobei die Aberration ausgewählt ist, um wenigstens eines aus einer Gruppe zu korrigieren, die Verzerrung, Schiefstellung, Astigmatismus, Coma, sphärische Aberration, Trefoil, Astigmatismus höherer Ordnungen, Coma und Sphärizität, Pentafoil, Tetrafoil und sphärische Aberrationen höherer Ordnung aufweist.

8. Intraokulare Linse (10) zum Kompensieren der optischen Effekte aufgrund kompressiver Verformung, die die Linsenoptik gemäß einem der Ansprüche 1 bis 7 und ein Paar von Haltebügeln (16) aufweist, die an die Linsenoptik gekoppelt sind.

9. Intraokulare Linse nach Anspruch 8, wobei die Haltebügel (16) eine erste Achse auf der Linsenoptik zwischen den Haltebügeln definieren, wobei eine zweite Achse auf der Linsenoptik in einem bestimmten Winkel in Bezug auf die erste Achse liegt, wobei die Linsenoptik eine dünnere Randdicke dort hat, wo der Rand die zweite Achse schneidet als dort, wo der Rand die erste Achse schneidet.

10. Intraokulare Linse nach Anspruch 8, wobei die erste Achse eine Kraftachse (f-f) aufweist und die zweite Achse eine Biegeachse (b-b) aufweist.

11. Verfahren zum Kompensieren eines optischen Effektes aufgrund von Verformung einer Linsenoptik, mit den Schritten:
Identifizieren (102) einer Aberration in dem Auge, für welches eine Korrektur erwünscht ist;
Ermitteln (108) eines erwarteten Kompressionsbetrages, der durch Implantation einer intraokularen Linse in eine Augenkammer verursacht wird; und
Konfigurieren der intraokularen Linse, dass sie eine erste Oberfläche (12) und eine zweite Oberfläche (14) hat, wobei die erste Oberfläche eine nicht komprimierte Geometrie der ersten Oberfläche hat und die zweite Oberfläche eine nicht komprimierte Geometrie der zweiten Oberfläche hat, wenn sie sich in einem nicht komprimierten Zustand befinden, wobei wenigstens eine von der ersten Oberfläche und der zweiten Oberfläche eine komprimierte Geometrie hat, wenn sie sich in einem komprimierten Zustand befinden, wobei die nicht komprimierte Geometrie der ersten Oberfläche und die nicht komprimierte Geometrie der zweiten Oberfläche so gewählt werden, dass sie wenigstens teilweise die durch die erwartete Kompression der Linse verursachten optischen Effekte kompensieren.

12. Verfahren nach Anspruch 11, welches ferner den Schritt der Erzeugung einer Aberration auf wenigstens einer von der ersten Oberfläche (12) oder der zweiten Oberfläche (14) aufweist, um eines von Astigmatismus, Coma oder sphärischer Aberration zu korrigieren.

13. Verfahren nach Anspruch 11, wobei Haltebügel (16) eine erste Achse auf der Linsenoptik zwischen den Bügeln definieren, die Linsenoptik eine zweite Achse in einem Winkel in Bezug auf die erste Achse definiert, und wobei sich die nicht komprimierte Geometrie von einer gewünschten Geometrie um die zweite Achse herum unterscheidet.

14. Verfahren nach Anspruch 11, wobei die Aberration auf etwa 0,17 D an der Stelle des Augenglases ausgebildet wird und bis zu etwa 0,25 D an der Ebene der intraokularen Linse annimmt.

15. Verfahren nach Anspruch 11, wobei die Ermittlung (108) des erwarteten Kompressionsbetrages, der durch Implantation der intraokularen Linse in die Kammer verursacht wird, eine Abschätzung eines Kompressionsbetrags der Linsenoptik umfasst, der der Kompression durch die Bügel (16) zuzuordnen ist.

16. Verfahren nach Anspruch 11, ferner mit dem Schritt der Formung der Linsenoptik mit einer oder mehr von der ersten Oberfläche (12) und der zweiten Oberfläche (14) mit einer asphärischen Kurve.

## Revendications

1. Optique à lentilles (11) destinée à une utilisation dans une lentille intraoculaire, comprenant :
une première surface (12) ayant une première géométrie de surface non compressée dans un état non compressé ; et
une seconde surface (14) ayant une seconde géométrie de surface non compressée dans un état non compressé ;
dans laquelle au moins une surface parmi la première surface et la seconde surface a une géométrie compressée lorsqu'elle est dans un état compressé, dans laquelle la première géométrie de surface non compressée et la seconde géométrie de surface non compressée sont sélectionnées pour au moins partiellement décaler les effets optiques, les effets optiques étant causés par la compression attendue de la lentille causée par l'implantation de la lentille dans la chambre d'un oeil.

2. Optique à lentilles selon la revendication 1, dans laquelle la géométrie compressée est due aux forces de compression exercées par un ou plusieurs éléments haptiques sur l'optique à lentilles.

3. Optique à lentilles selon la revendication 1, dans laquelle l'optique à lentilles (11) comprend une aberration.

4. Optique à lentilles selon la revendication 3, dans laquelle l'aberration est sélectionnée pour corriger un astigmatisme.

5. Optique à lentilles selon la revendication 3, dans laquelle l'aberration est basée sur une compression anticipée de 0,5 mm à 1,0 mm.

6. Optique à lentilles selon la revendication 3, dans laquelle l'aberration est sélectionnée pour corriger un coma.

7. Optique à lentilles selon la revendication 3, dans laquelle l'aberration est sélectionnée pour corriger au moins une aberration d'un groupe comprenant une distorsion, une inclinaison, un astigmatisme, un coma, une aberration sphérique, une aberration de tréfoil, un astigmatisme d'ordres plus élevés, un coma et une sphéricité, une aberration de pentafoil, une aberration de tétrafoil et des aberrations sphériques d'ordres plus élevés.

8. Lentille intraoculaire (10) pour décaler les effets optiques dus à une déformation par compression, comprenant l'optique à lentilles de l'une quelconque des revendications 1 à 7, et une paire d'éléments haptiques (16) couplés à l'optique à lentilles.

9. Lentille intraoculaire selon la revendication 8, dans laquelle les éléments haptiques (16) définissent un premier axe sur l'optique à lentilles entre les éléments haptiques, où un second axe sur l'optique à lentille fait un certain angle par rapport au premier axe, où l'optique à lentilles a une épaisseur de bord plus fine au niveau de l'intersection du bord et du second axe qu'au niveau de l'intersection du bord et du premier axe.

10. Lentille intraoculaire selon la revendication 8, dans laquelle le premier axe comprend un axe de force (f-f) et le second axe comprend un axe de flexion (b-b).

11. Procédé de décalage d'un effet optique dû à la déformation d'une optique à lentilles, comprenant les étapes consistant à :
identifier (102) une aberration dans l'oeil pour laquelle une correction est souhaitée ;
déterminer (108) une quantité attendue de compensation causée par l'implantation d'une lentille intraoculaire dans la chambre d'un oeil; et
configurer la lentille intraoculaire pour qu'elle ait une première surface (12) et une seconde surface (14), où la première surface a une première géométrie de surface non compressée et la seconde surface a une seconde géométrie de surface non compressée dans un état non compressé, où au moins une surface parmi la première surface et la seconde surface a une géométrie compressée lorsqu'elle est dans un état compressé, où la première géométrie de surface non compressée et la seconde géométrie de surface non compressée sont sélectionnées pour au moins partiellement décaler les effets optiques causés par la compression attendue de la lentille.

12. Procédé selon la revendication 11, comprenant en outre l'étape consistant à créer une aberration sur au moins une surface parmi la première surface (12) ou la seconde surface (14) pour corriger une aberration parmi un astigmatisme, un coma ou une aberration sphérique.

13. Procédé selon la revendication 11, dans lequel les éléments haptiques (16) définissent un premier axe sur l'optique à lentilles entre les éléments haptiques, l'optique à lentilles définissant un second axe faisant un certain angle par rapport au premier axe, la géométrie non compressée étant différente d'une géométrie souhaitée autour du second axe.

14. Procédé selon la revendication 11, dans lequel l'aberration est formée à environ 0,17 D à l'emplacement des lunettes et atteint environ 0,25 D au niveau du plan de la lentille intraoculaire.

15. Procédé selon la revendication 11, dans lequel la détermination (108) de la quantité attendue de compression causée par l'implantation de la lentille intraoculaire dans la chambre comprend d'estimer une quantité de compression de l'optique à lentilles attribuable à la compression par les éléments haptiques (16).

16. Procédé selon la revendication 11, comprenant en outre l'étape consistant à former l'optique à lentilles telle qu'elle ait une ou plusieurs surfaces, parmi la première surface (12) et la seconde surface (14), avec une courbure asphérique.
